# EUROPEAN PATENT APPLICATION

(11) **EP 4 115 893 A1**
(43) Date of publication of application: **11.01.2023**
(21) Application number: 21761700.0
(22) Date of filing: 01.03.2021
(51) Int. Cl.: A61K 35/74, A61K 39/00, A61K 45/06, A61P 35/00

(54) **ANTI-TUMOR COMPOSITION**

(30) Priority: 27.02.2020 CN 202010123922
(71) Applicant: SPH Sine Pharmaceutical Laboratories Co., Ltd, Shanghai 201206 (CN)
(72) Inventor: XU, Xiaofen, Shanghai 201206 (CN); YU, Hongjing, Shanghai 201206 (CN); LIANG, Xi, Shanghai 201206 (CN); SUN, Ningyun, Shanghai 201206 (CN); WEN, Bin, Shanghai 201206 (CN); GAO, Yuan, Shanghai 201206 (CN)
(74) Representative: Lahrtz, Fritz
(86) International application number: PCT/CN2021/078434
(87) International publication number: WO 2021/170137

(57) **Abstract**

Provided is an anti-tumor composition, comprising: (A) *Akkermansia muciniphila;* and (B) an immune checkpoint inhibitor, such as a PD-1 inhibitor, a PD-L1 inhibitor, a CTLA-4 inhibitor, etc. Also provided is use of *Akkermansia muciniphila* in preparing a drug for treating tumor.

## Description

### FIELD OF THE INVENTION

The present application relates to an anti-tumor composition, and more particularly to combined use of an *Akkermansia muciniphila* composition and an immune checkpoint inhibitor.

### RELATED ART

Curing cancer has always been a major challenge in the medical field, and traditional treatment methods such as chemotherapy and radiotherapy can cause greater side effects and pain to patients. In recent years, immunotherapy for cancer has shown amazing efficacy in hematologic tumors and some solid tumors with fewer side effects on patients, which gives great hope for curing cancer in humans.

Under normal circumstances, there are antigens on the surface of tumor cells that can be recognized by human immune T cells. And the human immune system is able to recognize and kill the tumor cells. However, in order to survive and grow, the tumor cells will adopt various ways to avoid recognition and killing by the immune system. Tumor immunotherapy is an anti-tumor immune response that identifies and destroys such trick of the tumor cells to restore the body to normal, so as to control and eliminate a wide range of tumors. Tumor immunotherapy includes five main types: (1) therapeutic antibodies; (2) cancer vaccines; (3) cell therapy, i.e., CAR-T therapy; (4) immunomodulators; and (5) immune checkpoint inhibitors.

The activity of the human immune system is regulated by co-stimulatory molecules, and the co-stimulatory molecules are immune checkpoints. When antigen recognition occurs, other molecules interact with immune cells and target cell surface molecules, which in turn determine the balance of interactions. If the signal is largely positive, the immune cells will be activated and attack the antigen presented by the target cell. Conversely, if the signal is negative, the immune cells will be inactivated. This inactivation is sometimes permanent, and the antigen is recognized as a normal/self-antigen. Cancer-related immune checkpoints that have been relatively well established include CTLA-4, PD-1 and PD-L1. Immune checkpoint inhibitors are monoclonal antibody medicaments developed to target the corresponding immune checkpoints. The main role of the immune checkpoint inhibitors is to block the interaction between the tumor cells expressing the immune checkpoints and the immune cells, thus blocking the inhibitory effect of the tumor cells on the immune cells. Currently widely used immune checkpoint inhibitors include monoclonal antibodies targeting programmed death protein 1 (PD-1) and its ligand (PD-L1). PD-1 antibodies are very effective in blocking advanced melanoma, non-small cell lung cancer and renal cell carcinoma, and have made a major breakthrough in the treatment of tumors. However, due to the complex pathogenesis of tumors, the large individual differences for patients, and the influence of environmental factors, the immune checkpoint inhibitors can only be effective in about 25% of patients at present.

More and more research suggests that gut microbes are also an important factor affecting the effectiveness of cancer immunotherapy. In 2015, Marie Vétizou et al. found that tumors inoculated into antibiotic-treated or germ-free mice did not respond to CTLA-4 inhibitors, and that the anti-tumor effects of CTLA-4 inhibitors were restored when *Bacteroides fragilis* was administered to the mice. In 2017, a study by Routy B et al. showed that anti-PD-1 inhibitors in combination with certain specific gut microbiota significantly improved the response of tumor patients and significantly improved the mean progression-free survival (PFS) of the patients. In summary, gut microbes have great potential for use in improving the efficacy of immune checkpoint inhibitors. Therefore, there is a need in the art to develop a new anti-tumor composition with better anti-tumor effects, especially with better anti-tumor effects on specific tumors.

### SUMMARY

The inventors of the present application have found that the combined use of an *Akkermansia muciniphila* composition and an immune checkpoint inhibitor is effective in inhibiting various tumors including, but not limited to, colon cancer, lung cancer, breast cancer, melanoma, kidney cancer, urothelial cancer, and the like.

One aspect of the present application provides an anti-tumor composition, which includes:
(A) *Akkermansia muciniphila;* and
(B) an immune checkpoint inhibitor.

In one embodiment of the present application, the *Akkermansia muciniphila* is *Akkermansiamuciniphila* SSYD-3 with the deposit number of CGMCC No. 14764.

In one embodiment of the present application, the *Akkermansia muciniphila* is combined with other bacteria (such as *Bifidobacterium, Lactobacillus, Clostridium, Enterococcus faecium, Prevotella stercorea,* and *Ruminococcus*).

In one embodiment of the present application, the *Akkermansia muciniphila* and the immune checkpoint inhibitor are mixed together to prepare a single preparation, or physically separated and used separately.

In one embodiment of the present application, the *Akkermansia muciniphila* is administered orally.

In one embodiment of the present application, the anti-tumor composition further comprises antibiotics.

In one embodiment of the present application, the tumor is selected from colon cancer, lung cancer, breast cancer, melanoma, kidney cancer or urothelial cancer.

Another aspect of the present application provides use of *Akkermansia muciniphila* in the manufacture of a medicament for treating tumors.

In one embodiment of the present application, the *Akkermansia muciniphila* is used in combination with an immune checkpoint inhibitor.

In one embodiment of the present application, the use is to improve the effect of the immune checkpoint inhibitor in suppressing tumors, preferably, the tumors are selected from colon cancer, lung cancer, breast cancer, melanoma, kidney cancer or urothelial cancer.

### BRIEF DESCRIPTION OF THE DRAWINGS

Figure 1 depicts the effect of gut microbiota disruption caused by antibiotic treatment on the therapeutic effect of anti-m PD-1 in MC38 tumor-bearing mice.
Figure 2 depicts the therapeutic effects of injections of anti-m PD-1 alone and in combination with *Akkermansia muciniphila* SSYD-3 on MC38 tumors.
Figure 3 depicts the number of tumor-infiltrating CD8 T cells in MC38 tumor-bearing mice when anti-m PD-1 is injected alone and used in combination with *Akkermansia muciniphila* SSYD-3.
Figure 4 depicts the mean number of CD3 cells in MC38 tumor-bearing mice when anti-m PD-1 is injected alone and used in combination with *Akkermansia muciniphila* SSYD-3.
Figure 5 depicts the mean number of Treg cells in MC38 tumor-bearing mice when anti-m PD-1 is injected alone and used in combination with *Akkermansia muciniphila* SSYD-3.
Figure 6 depicts the therapeutic effects of injections of anti-m PD-1 alone and in combination with *Akkermansia muciniphila* SSYD-3 on 4T1 tumors.
Figure 7 depicts the therapeutic effects of injections of anti-m PD-1 alone and in combination with *Akkermansia muciniphila* SSYD-3 on LLC1 tumors.

### DETAILED DESCRIPTION

In the present invention, unless otherwise specified, percent (%) or parts refer to percent by weight or parts by weight relative to the composition.

In the present invention, unless otherwise specified, various components or exemplary components thereof involved herein may be combined with each other to form a new technical solution.

In the present invention, unless otherwise specified, all embodiments or exemplary embodiments mentioned in this specification may be combined with each other to form a new technical solution.

In the present invention, unless otherwise specified, all technical features or exemplary technical features mentioned in this specification may be combined with each other to form a new technical solution.

In the present invention, the sum of the contents of the components in the composition is 100%, if not stated to the contrary.

In the present invention, the sum of the parts of the components in the composition may be 100 parts by weight, if not stated to the contrary.

In the present invention, the numerical range "a-b" means an abbreviated representation of any combination of real numbers between a and b, where both a and b are real numbers, unless otherwise specified. For example, the numerical range "0-5" means that all real numbers between "0-5" have been listed herein, and "0-5" is only an abbreviated representation of the combination of these values.

In the present invention, the integer numerical range "a-b" means an abbreviated representation of any combination of integers between a and b, where both a and b are integers, unless otherwise specified. For example, the integer numerical range "1-N" means 1, 2, ...N, where N is an integer.

In the present invention, "combination thereof" means a multi-component mixture of said components, such as two-, three-, four- and multi-component mixture with the largest possible number of components, unless otherwise specified.

The term "a" as used in this specification means "at least one", if not specifically indicated.

The basis for the percentages (including weight percentages) as described in the present invention is the total weight of said composition, if not specifically indicated.

The "scope" disclosed herein is in the form of lower and upper limits. There can be one or more lower limits, and one or more upper limits, respectively. The given range is defined by selecting a lower limit and an upper limit. The selected lower and upper limits define the boundaries of the special range. All ranges that can be defined in this manner are inclusive and combinable, i.e., any lower limit can be combined with any upper limit to form a range. For example, ranges 60-120 and 80-110 are listed for specific parameters, with the understanding that ranges 60-110 and 80-120 are also to be expected. In addition, if the minimum range values 1 and 2 are listed, and if the maximum range values 3, 4, and 5 are listed, the following ranges can all be expected: 1-3, 1-4, 1-5, 2-3, 2-4, and 2-5.

As used herein, the proportions or weights of the components refer to dry weight unless otherwise stated.

As used herein, "constant" means a change within ±10%, preferably within ±5%, more preferably within ±2%, and finally within ±1%, unless otherwise stated.

As used herein, *Akkermansia muciniphila* includes active ingredients derived from *Akkermansia muciniphila*, or a strain having at least 97% sequence similarity with *Akkermansia muciniphila* identified by sequencing.

### Akkermansia muciniphila

The *Akkermansia muciniphila* described in the present application can be obtained from commercially available sources or according to prior art. For example, the Chinese invention patent application with application number CN201380070847.0 and publication number CN 104918626 A discloses "Use of *Akkermansia* for treatment of metabolic disorders", which discloses *Akkermansia muciniphila* ATCC BAA835.

In a preferred embodiment of the present application, the *Akkermansiamuciniphila* strain is named as *Akkermansiamuciniphila* SSYD-3 with the deposit number of CGMCC No. 14764.

The *Akkermansiamuciniphila* SSYD-3 (strain W03 for short) of the present invention has been deposited in the Chinese General Microbiological Culture Collection Center (CGMCC) on September 29, 2017 at Building 3, NO.1 West Beichen Road, Chaoyang District, Beijing 100101, with the deposit number of CGMCC No. 14764, culture name of *Akkermansiamuciniphila* SSYD-3, and taxonomic name of *Akkermansiamuciniphila.*

The *Akkermansia muciniphila* strain can be formulated into various suitable dosage forms, such as oral solution, tablet, capsule, orally disintegrating tablet, lyophilized powder and the like. In a preferred embodiment of the present application, the dosage form is a capsule. In another preferred embodiment of the present application, the dosage form is a tablet. The dosage form is preferably a lyophilized bacterial preparation, in which the number of viable bacteria is preferably 10¹⁰ CFU/g. Among which, the effective dose means that a solid viable bacterial preparation prepared by using *Akkermansia muciniphila* as the main pharmaceutical active ingredient contains a total number of viable bacteria of 10⁶-10¹⁴ CFU/g. Among which, the effective dose means that a liquid viable bacterial preparation prepared by using *Akkermansia muciniphila* as the main pharmaceutical active ingredient contains a total number of viable bacteria of 10⁶-10¹⁴ CFU/mL.

In a preferred embodiment of the present application, the *Akkermansia muciniphila* can also be used in combination with other bacterial strains (including but not limited to *Bifidobacterium, Lactobacillus, Clostridium, Enterococcus faecium, Prevotella stercorea, Ruminococcus,* and the like).

### Immune checkpoint inhibitor

The immune checkpoint inhibitor used in the present application can be any commercially available product, such as PD-1 inhibitor, PD-L1 inhibitor, CTLA-4 inhibitor, and the like. In a preferred embodiment, the immune checkpoint inhibitor includes Keytruda, Tecentriq, Nivolumab Injection, Bavencio (Avelumab), and Tuoyi (Toripalimab Injection).

### Anti-tumor composition

In the present application, the *Akkermansia muciniphila* strain and the immune checkpoint inhibitor can be mixed together to prepare a single preparation for combined use, or can be physically separated and used separately. In one embodiment of the present application, the *Akkermansia muciniphila* strain and the immune checkpoint inhibitor are physically separated and used separately. In another embodiment of the present application, the *Akkermansia muciniphila* strain can be administered to a patient prior to the administration of the immune checkpoint inhibitor. In general, the *Akkermansia muciniphila* strain can be administered to the patient in any suitable manner (including, but not limited to, oral administration, injection, and the like). The immune checkpoint inhibitor can be administered to the patient in any suitable manner (including, but not limited to, oral administration, injection, and the like). The method for using the *Akkermansia muciniphila* strain or the immune checkpoint inhibitor is routine in the art, and can be directly determined by those of ordinary skill in the art according to the description in the specification in combination with the prior art.

In the present application, the anti-tumor composition may further include antibiotics. The antibiotic can be any suitable antibiotic, such as, but not limited to, quinolone antibiotics, β-lactam antibiotics, macrolides, aminoglycoside antibiotics, and the like. In a preferred embodiment of the present application, the antibiotics include but are not limited to β-lactam antibiotics (such as penicillin, ampicillin, carbenicillin, methicillin, oxacillin, dicloxacillin, flucloxacillin, cefradine, cefotaxime, cefalexin, ceftriaxone, cefpirome, cefixime, cefditoren pivoxil, cefdinir, ceftibuten, cefpodoxime proxetil, imipenem, aztreonam, cefminox sodium, biapenem, imipenem, meropenem, and the like); macrolide antibiotics (such as erythromycin, albomycin, roxithromycin, erythromycin ethylsuccinate, azithromycin, clarithromycin, acetyl spiramycin, meleumycin, medemycin, josamycin, telithromycin, and the like); aminoglycoside antibiotics (streptomycin, gentamicin, arbekacin, amikacin, and the like); quinolone antibiotics (such as ciprofloxacin, levofloxacin, norfloxacin, and the like); other antibiotics and antibacterial drugs (such as tetracyclines, chloramphenicols, lincomycin, rifamycins such as rifapentini, polypeptides such as vancomycin, sulfonamides such as sulfamethoxazole, metronidazoles, and the like); antifungal drugs (such as amphotericin, griseofulvin, Daktarin, and the like); and anti-tumor antibiotics (such as mitomycin, actinomycin D, bleomycin, doxorubicin, and the like) and the like.

In the present application, the antibiotic may be packaged separately from other components and administered separately.

In a preferred embodiment of the present application, the anti-tumor composition further includes instructions describing administration of *Akkermansia muciniphila* strain for a period of time (e.g., 1-10 days), followed by administration of the PD-1 inhibitor for a period of time (e.g., 10-100 days). Preferably, a dose range of *Akkermansia muciniphila* strain is 10⁴-10¹³ CFU/person/day, and a dose range of the immune checkpoint inhibitor is 0.5-10 mg/kg.

The instructions also describe the administration of antibiotics for a period of time (e.g., 1-3 days) prior to the administration of *Akkermansia muciniphila* strain and the immune checkpoint inhibitor. Preferably, a dose range of the antibiotics is 1-500 mg/kg.

The anti-tumor composition of the present application can effectively treat various tumors, especially (but not limited to) colon cancer, lung cancer, breast cancer, melanoma, kidney cancer, urothelial cancer and the like.

Another aspect of the present application provides use of *Akkermansia muciniphila* strain in the manufacture of a medicament for treating tumors.

In the present application, the *Akkermansia muciniphila* strain can be combined with immune checkpoint inhibitors to inhibit the growth of tumors (such as colon cancer, lung cancer, breast cancer, melanoma, kidney cancer, urothelial cancer, and the like). The inventors found that the effect of the immune checkpoint inhibitors in inhibiting tumor growth is limited when antibiotics are administered to patients, however, administration of *Akkermansia muciniphila* strain prior to or concurrently with the immune checkpoint inhibitors may effectively enhance the tumor suppressive effect of the immune checkpoint inhibitors.

Another aspect of the present application provides use of *Akkermansia muciniphila* strain in treatment of tumors. In one embodiment of the present application, the use includes inhibiting tumor growth. Preferably, the *Akkermansia muciniphila* strain may be used in combination with an immune checkpoint inhibitor. Typically, the tumors include, but not limited to, colon cancer, lung cancer, breast cancer, melanoma, kidney cancer, urothelial cancer, and the like.

This application is described in detail below with reference to the embodiments, but the scope of this application is not limited thereto.

The raw materials used in the examples are as follows:
SPF grade male C57BL/6J mice aged 6-8 weeks (20-26 g) are from Jiangsu Jicui Yaokang Biotechnology Co., Ltd. with a quality certificate number of 201805120. The rearing conditions are as follows: the temperature is controlled at (23±3)°C, the humidity is 40-70%, and the mice are allowed to eat and drink freely.

Anti-PD-1 inhibitor is purchased from Yikang (Beijing) Pharmaceutical Technology Co., Ltd. at a concentration of 7.09 mg/mL, and stored at 2-8°C in the dark. An appropriate amount of phosphate buffered saline (PBS) is added and well mixed to a specified concentration.

Ampicillin is purchased from Anhui Anfengtang Animal Medicine Industry Co., Ltd., Streptomycin is purchased from Solarbioy, and Colistin Sulfate Soluble Powder is purchased from Shandong Luxi Animal Medicine Share Co., Ltd.

MC38 tumor cells are purchased from Prutine Biotechnology (Beijing) Co., Ltd. and are cultured with a DMEM medium containing inactivated 10% fetal bovine serum, 100 U/mL of penicillin, 100 µg/mL of streptomycin and 2 mM of glutamine in a 5% CO₂ incubator at 37°C. The cells are split into flasks and passaged every 3 days or so when the incubator is full of cells. The tumor cells in the logarithmic growth phase are used for tumor inoculation in vivo.

Mouse breast cancer 4T1 cells (ATCC, product number: CRL-2539), and LLC1 lung cancer cells (purchased from Shanghai Institutes for Biological Sciences).

### Measurement of tumor volume:

Tumor volume: a vernier caliper is used to measure the tumor volume 3 times a week, and the long and short diameters of the tumor are measured. The calculation formula for tumor volume is: volume=0.5 × long diameter × short diameter².

### Example 1: Lyophilized bacteria agent of Akkermansia muciniphila CGMCC No.14764

### Medium

Separation medium: 0.4 g KH₂PO₄; 0.53 g Na₂HPO₄; 0.3 g NH₄Cl; 0.3 g NaCl; 0.1 g MgCl₂·6H₂O; 0.11 g CaCl₂; 0.5 mg resazurin; 4 g NaHCO₃; 0.25 g Na₂S·7-9H₂O; and 0.25% mucin.

Fermentation medium 1: Brain Heart Infusion Broth (Brain Heart Extract Powder, BHI)

Fermentation medium 2: 5 g tryptone, 5 g soya peptone, 5 g beef powder, 5 g yeast powder, 4 g glucose, 3 g NaCl, 1.5 g MgSO₄, Na₂HPO₄, KH₂PO₄, 1 L water, pH=7.2.

The separation and fermentation media were autoclaved at 121°C for 15 min for later use.

### Isolation and identification of strains

1. The prepared mucin separation medium was placed in a serum bottle (filling volume of 10/30 mL)
2. 0.5 g of healthy adult feces were placed in sterile phosphate buffered saline (PBS) and dispersed evenly. The feces were sequentially and gradiently diluted 10-fold in the PBS solution, and 1 mL of the dilution was inoculated into a serum bottle of mucin separation medium
3. The mucin serum bottle was placed at 37°C and cultured under an anaerobic environment for 48 h
4. The mucin fermentation broth at the highest dilution gradient with visible turbidity was taken and diluted to 10⁻⁶-10⁻⁹ with PBS buffer
5. The dilution was coated on the Brain Heart Infusion agar medium, cultured for 24-48 h, and colonies with a colony size of 1 mm were picked, purified and cultured for bacterial 16S rDNA identification

Results: based on the morphological characteristics, physiological and biochemical characteristics of the colony as well as the amplification of bacterial 16SrDNA, the 16S rDNA sequence of W03 bacteria was shown in sequence listing 1. After comparing homology between the sequenced sequence and the uploaded 16S rDNA gene sequence in GenBank, the isolated bacterium was identified as *Akkermansiamuciniphila,* named as *Akkermansiamuciniphila* SSYD-3 with the deposit number of CGMCC No. 14764. The alignment similarity with the existing sequence was up to 99%.

### Akkermansia muciniphila fermentation

1. *Akkermansia muciniphila* was inoculated into Brain Heart Infusion agar via a three sector streak method, and cultured under an anaerobic environment at 37°C for 48 h.
2. A single colony of *Akkermansia muciniphila* was picked from the agar plate, inoculated into 15 mL of Brain Heart Infusion liquid medium, and cultured under an anaerobic environment at 37°C for 24 h.
3. 10 mL of fermentation broth was transferred from a 15 mL anaerobic tube to 200 mL of Brain Heart Infusion liquid medium, and cultured under an anaerobic environment at 37°C for 24 h.
4. 200 mL of *Akkermansia muciniphila* fermentation broth was inoculated into the Brain Heart Infusion liquid medium, and cultured under an anaerobic environment at 37°C for 24 h, where the filling volume of a 5 L fermentation tank was 4 L, the anaerobic gas was a mixed binary gas of nitrogen and carbon dioxide (N2:CO2=9:1), and the stirring speed was 100 r/min.

### Lyophilized bacteria agent of Akkermansia muciniphila CGMCC No. 14764

1. Freeze-drying protective agent: trehalose, sucrose, milk powder.
2. The above-obtained fermentation broth was centrifuged at 8000 R for 20 min, and the cells were collected.
3. The cells obtained by centrifugation of *Akkermansia muciniphila* were mixed with the freeze-drying protective agent and water, so that the mass percentages of trehalose, sucrose and milk powder in the solution before freeze-drying were 5%, 5% and 10% respectively, and lyophilized. Lyophilized bacterial powder was obtained, and the number of viable bacteria of *Akkermansia muciniphila* strain with the deposit number of CGMCC No. 14764 in the bacterial powder was 10¹⁰ CFU/g.

### Example 2

48 male C57BL/6J mice aged 6-8 weeks (20-26 g, SPF grade) were randomly divided into 4 groups based on body weight after acclimatization for one week, with 12 mice in each group: group 1 (no antibiotic treatment, blank, *i.p.),* group 2 (no antibiotic treatment, anti-mPD-1, 10 mg/kg, *i.p.),* group 3 (antibiotic treatment, blank, *i.p.),* and group 4 (antibiotic treatment, anti-mPD-1, 10 mg/kg, *i.p.).* Antibiotic treatment was done by using broad-spectrum antibiotics Ampicillin (1 mg/mL) + colistin (1 mg/mL) + streptomycin (5 mg/mL) in drinking water for 5 days. Mice in all groups were subcutaneously inoculated with MC38 tumor cells resuspended in phosphate buffered saline (PBS) at a concentration of 1×10⁷ cells/mL to the right flank of experimental animals at 100 µL/mouse. Mice in groups 2 and 4 were injected with anti-mPD-1 on day 4 after tumor cell inoculation, once every 4 days, for a total of 4 injections. Mice were euthanized on day 19 after tumor inoculation, and the tumor of the mice in each group was picked off.

MC38 tumor cells were purchased from Prutine Biotechnology Co., Ltd. and were cultured with a DMEM medium containing inactivated 10% fetal bovine serum, 100 U/mL of penicillin, 100 µg/mL of streptomycin and 2 mM of glutamine in a 5% CO₂ incubator at 37°C. The cells were split into flasks and passaged every 3 days or so when the incubator is full of cells. The tumor cells in the logarithmic growth phase were used for tumor inoculation in vivo.

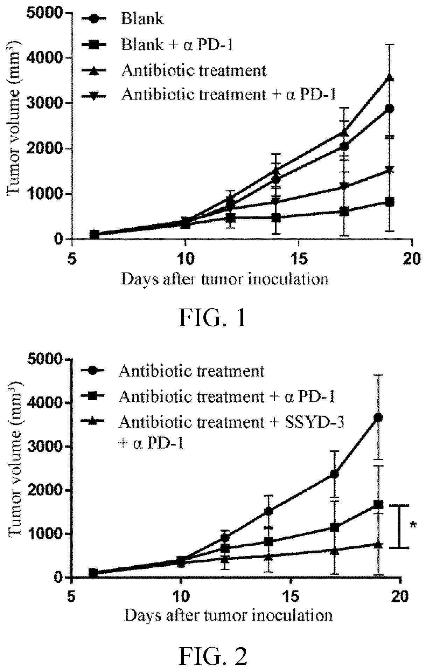

The experimental results were shown in Figure 1. The results showed that after antibiotic treatment disrupted gut microbiota of the mice, tumor growth of the mice was accelerated, the tumor volume was increased, and the anti-tumor effect of anti-m PD-1 was reduced, which indicated that the gut microbiota affected the tumor growth of the mice and the anti-tumor effect of anti-m PD-1.

### Example 3

48 male C57BL/6J mice aged 6-8 weeks (20-26 g, SPF grade) were randomly divided into 4 groups based on body weight after acclimatization for one week: group 1 (no antibiotic treatment, blank, *i.p.),* group 2 (antibiotic treatment, blank, *i.p.*), group 3 (antibiotic treatment, anti-mPD-1, 10 mg/kg, *i.p.*), and group 4 (antibiotic treatment, *Akkermansia muciniphila* SSYD-3, *p.o*., anti-mPD-1, 10 mg/kg, *i.p.*)*.* Antibiotic treatment was done by using broad-spectrum antibiotics Ampicillin (1 mg/mL) + colistin (1 mg/mL) + streptomycin (5 mg/mL) in drinking water for 5 days. Mice in group 4 were given with lyophilized samples of *Akkermansia muciniphila* SSYD-3 by gavage at a concentration of 10×10⁸ CFU/mouse/day. After 2 weeks of continuous gavage, all groups were subcutaneously inoculated with MC38 tumor cells resuspended in PBS at a concentration of 1×10⁷ cells/mL to the right flank of experimental animals at 100 µL/mouse. Mice in groups 3 and 4 were injected with anti-mPD-1 on day 4 after tumor cell inoculation, once every 4 days, for a total of 4 injections. Mice were weighed on day 19 after tumor inoculation and then euthanized, and the weights of the tumors of the mice were weighed and recorded.

MC38 tumor cells were purchased from Prutine Biotechnology Co., Ltd. and were cultured with a DMEM medium containing inactivated 10% fetal bovine serum, 100 U/mL of penicillin, 100 µg/mL of streptomycin and 2 mM of glutamine in a 5% CO₂ incubator at 37°C. The cells were split into flasks and passaged every 3 days or so when the incubator is full of cells. The tumor cells in the logarithmic growth phase were used for tumor inoculation in vivo.

The test results were shown in Figure 2. Compared with the anti-mPD-1 alone treatment group, the combined treatment of the strain and the anti-mPD-1 significantly inhibited the tumor growth and significantly reduced the tumor volume. The results showed that *Akkermansia muciniphila* SSYD-3 could effectively improve the therapeutic effect of anti-mPD-1. P<0.05 indicates a significant difference.

### Example 4

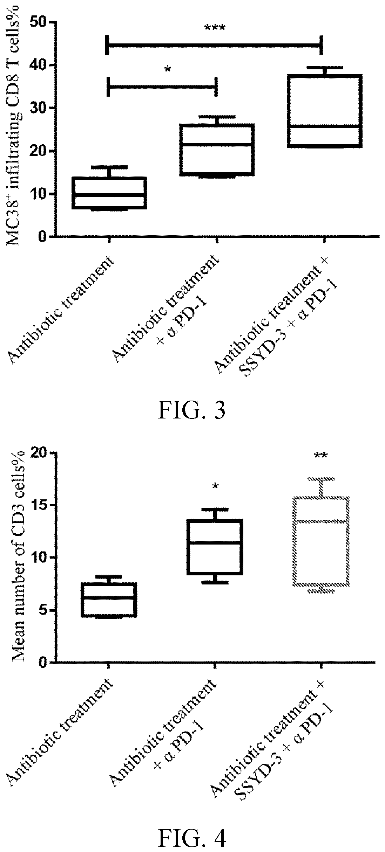

The mice in Example 3 were euthanized on day 19 after tumor inoculation, and flow cytometry (Aisen Biotechnology Co., Ltd, NovoCyte 3130) was used to detect CD3, CD4, CD8, FOXP3, CD25, CXCR3, Gata3, Granzyme B, CD69, PD-1, CTLA-4 and CD11b, MHC II, CD206, CD40, CSF1R, PD-L1, and Gr-1 in tumor cells. Immune factor analysis included TNF-α, IL-17, IL-13, IL-12p70, IL-10, IL-6, IL-5, IL-4, IL-2, IL-1b, IFNy, GM-CSF, G-CSF, M-CSF, MIG, IP-10, MIP1b and MAC-1. In antibiotic-treated mice, the treatment result of anti-m PD-1 alone as well as the combined treatment result of *Akkermansia muciniphila* SSYD-3 and anti-m PD-1 were shown in Figure 3. The results showed that the combined treatment of *Akkermansia muciniphila* SSYD-3 and the anti-m PD-1 injection could significantly increase the number of tumor-infiltrating CD8 T cells, which indicated that *Akkermansia muciniphila* SSYD-3 enhanced the anti-tumor effect of anti-mPD-1.

### Example 5:

The mice in Example 3 were euthanized on day 19 after the treatment of anti-m PD-1 alone and the combined treatment of *Akkermansia muciniphila* SSYD-3 and anti-m PD-1, and flow cytometry (Aisen Biotechnology Co., Ltd, NovoCyte 3130) was used to detect CD3, CD4, CD8, FOXP3, CD25, CXCR3, Gata3, Granzyme B, CD69, PD-1, CTLA-4 and CD11b, MHC II, CD206, CD40, CSF1R, PD-L1, and Gr-1 in tumor cells. Immune factor analysis includes TNF-α, IL-17, IL-13, IL-12p70, IL-10, IL-6, IL-5, IL-4, IL-2, IL-1b, IFNy, GM-CSF, G-CSF, M-CSF, MIG, IP-10, MIP1b and MAC-1. The results of immune cell changes in the mice were shown in Figure 4. The results showed that compared with the injection of anti-m PD-1 alone, the combined treatment of anti-m PD-1 and *Akkermansia muciniphila* SSYD-3 significantly increased the level of CD3 cells in tumor tissues and enhanced the body's immunity. Compared with the antibiotic treatment group in the figures, P<0.05 indicates a significant difference, ^{∗}P<0.05, ^{∗∗}P<0.01.

### Example 6

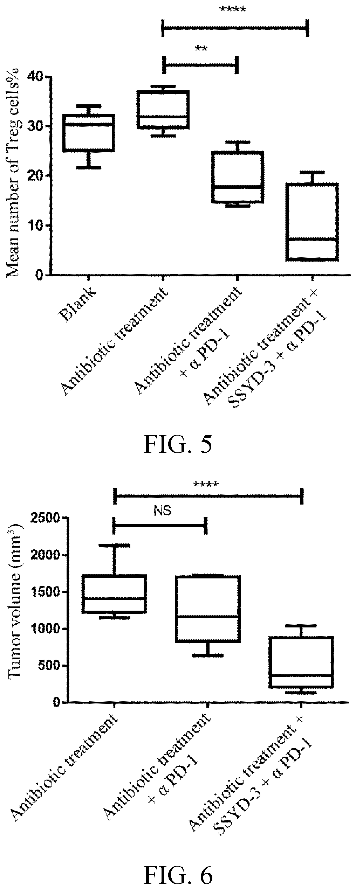

The mice in Example 3 were euthanized on day 19 after the treatment of anti-m PD-1 alone and the combined treatment of *Akkermansia muciniphila* SSYD-3 and anti-m PD-1, and flow cytometry (Aisen Biotechnology Co., Ltd, NovoCyte 3130) was used to detect CD3, CD4, CD8, FOXP3, CD25, CXCR3, Gata3, Granzyme B, CD69, PD-1, CTLA-4 and CD11b, MHC II, CD206, CD40, CSF1R, PD-L1, and Gr-1 in tumor cells. Immune factor analysis includes TNF-α, IL-17, IL-13, IL-12p70, IL-10, IL-6, IL-5, IL-4, IL-2, IL-1b, IFNy, GM-CSF, G-CSF, M-CSF, MIG, IP-10, MIP1b and MAC-1. The experimental results were shown in Figure 5. The number of Treg cells in the tumor tissue of the mice after antibiotic treatment increased slightly, but there was no significant difference. The number of Treg cells in the antibiotic treatment group treated with anti-mPD-1 was significantly decreased (P<0.01), and the combined treatment of *Akkermansia muciniphila* SSYD-3 and anti-mPD-1 also significantly decreased the number of Treg cells in the mice (P<0.001). The above results showed that the injection of anti-mPD-1 alone partially relieved the immune suppression system in the mice, and the combined use with *Akkermansia muciniphila* SSYD-3 further relieved the immune suppression in the mice and restored the immune activity and anti-tumor effect of the organism. Compared with the antibiotic treatment group, P<0.05 indicates a significant difference, ^{∗∗}P<0.01, ^{∗∗∗}P<0.001.

### Example 7

36 male C57BL/6J mice aged 6-8 weeks (20-26 g, SPF grade) were randomly divided into 3 groups based on body weight after acclimatization for one week: group 1 (antibiotic treatment, blank, *i.p.*), group 2 (antibiotic treatment, anti-mPD-1, 10 mg/kg, *i.p.),* and group 3 (antibiotic treatment, *Akkermansia muciniphila* SSYD-3, p.o., anti-mPD-1, 10 mg/kg, *i.p.*)*.* The mice in each group were administered with broad-spectrum antibiotics Ampicillin (1 mg/mL) + colistin (1 mg/mL) + streptomycin (5 mg/mL) in drinking water for 5 days. Then, group 3 was given with lyophilized samples of *Akkermansia muciniphila* SSYD-3 by gavage at a concentration of 1.0×10⁸ CFU/mouse/day. After 2 weeks of continuous gavage, the mice in all groups were subcutaneously inoculated with 4T1 tumor cells resuspended in PBS at a concentration of 1×10⁷ cells/mL to the right flank of experimental animals at 100 µL/mouse. Mice in groups 2 and 3 were injected with anti-mPD-1 on day 4 after tumor cells inoculation, once every 4 days, for a total of 4 injections. The mice were measured for tumor volume on day 19 after tumor inoculation, then the mice in each group were euthanized and tumor tissues were picked off.

The results were shown in Figure 6. In a 4T1 breast cancer model insensitive to anti-mPD-1 treatment, the combined treatment of *Akkermansia muciniphila* SSYD-3 and anti-mPD-1 significantly enhanced the therapeutic effect of anti-mPD-1 on 4T1 breast cancer (P<0.0001), which indicated that *Akkermansia muciniphila* SSYD-3 could enhance the response to anti-mPD-1 in the organism and widen the application scope of anti-mPD-1 for tumor treatment.

### Example 8:

36 male C57BL/6J mice aged 6-8 weeks (20-26 g, SPF grade) were randomly divided into 3 groups based on body weight after acclimatization for one week: group 1 (antibiotic treatment, blank, *i.p.*), group 2 (antibiotic treatment, anti-mPD-1, 10 mg/kg, *i.p.*), and group 3 (antibiotic treatment, *Akkermansia muciniphila* SSYD-3, p.o., anti-mPD-1, 10 mg/kg, *i.p.*)*.* The mice in each group were administered with broad-spectrum antibiotics Ampicillin (1 mg/mL) + colistin (1 mg/mL) + streptomycin (5 mg/mL) in drinking water for 5 days. The mice in group 3 were given with lyophilized samples of *Akkermansia muciniphila* SSYD-3 by gavage at a concentration of 1.0×10⁸ CFU/mouse/day. After 2 weeks of continuous gavage, all groups were subcutaneously inoculated with LLC1 lung cancer cells resuspended in PBS at a concentration of 1×10⁵ cells/mL to the right flank of experimental animals at 100 µL/mouse. Mice in groups 2 and 3 were injected with anti-mPD-1 on day 4 after tumor cell inoculation, once every 4 days, for a total of 4 injections. The mice were measured for tumor volume on day 19 after tumor inoculation, then the mice in each group were euthanized and tumor tissues were picked off.

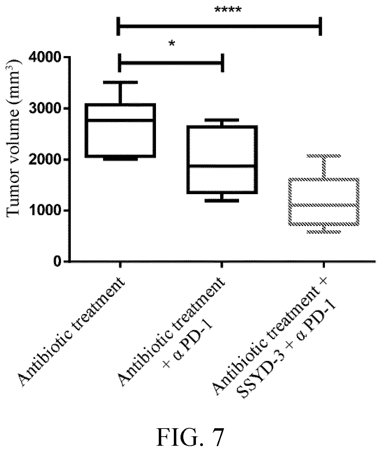

As shown in Figure 7, in the LLC1 lung cancer model less responsive to anti-mPD-1, compared to treatment of anti-mPD-1 alone, antibiotic-treated mice treated with anti-mPD-1 in combination with *Akkermansia muciniphila* SSYD-3 had significantly reduced tumor volume (P<0.0001), which indicated that *Akkermansia muciniphila* SSYD-3 significantly improved the response of LLC1 lung cancer to anti-mPD-1 and widened the application scope of anti-mPD-1 for tumor treatment.

## Claims

1. An anti-tumor composition, comprising:
(A) *Akkermansia muciniphila;* and
(B) an immune checkpoint inhibitor.

2. The anti-tumor composition of claim 1, wherein the *Akkermansia muciniphila* is *Akkermansiamuciniphila* SSYD-3 with the deposit number of CGMCC No. 14764.

3. The anti-tumor composition of claim 1 or 2, wherein the *Akkermansia muciniphila* is combined with other bacteria (such as *Bifidobacterium, Lactobacillus, Clostridium, Enterococcus faecium, Prevotella stercorea, Ruminococcus).*

4. The anti-tumor composition of claim 1 or 2, wherein the *Akkermansia muciniphila* and the immune checkpoint inhibitor are mixed together to prepare a single preparation, or physically separated and used separately.

5. The anti-tumor composition of claim 1 or 2, wherein the *Akkermansia muciniphila* is administered orally.

6. The anti-tumor composition of claim 1 or 2, further comprising antibiotics.

7. The anti-tumor composition of claim 1 or 2, wherein the tumor is selected from colon cancer, lung cancer, gastric cancer, liver cancer, head and neck cancer, cervical cancer, breast cancer, lymphoma, breast cancer, melanoma, kidney cancer or urothelial cancer.

8. Use of *Akkermansia muciniphila* in the manufacture of a medicament for treating tumors.

9. The use of claim 8, wherein the *Akkermansia muciniphila* is used in combination with an immune checkpoint inhibitor.

10. The use of claim 8 or 9, wherein the use is to improve the effect of the immune checkpoint inhibitor in suppressing tumors, preferably, the tumors are selected from colon cancer, lung cancer, gastric cancer, liver cancer, head and neck cancer, cervical cancer, breast cancer, lymphoma, breast cancer, melanoma, kidney cancer or urothelial cancer.
